# EUROPEAN PATENT APPLICATION

(11) **EP 3 430 932 A1**
(43) Date of publication of application: **23.01.2019**
(21) Application number: 16893856.1
(22) Date of filing: 15.03.2016
(51) Int. Cl.: A43B 3/00, G01G 19/44

(54) **SHOE AND CONTROL METHOD THEREOF**

(71) Applicant: Shenzhen Royole Technologies Co., Ltd., Shenzhen, Guangdong 518052 (CN)
(72) Inventor: YANG, Songling, Shenzhen Guangdong 518052 (CN); ZHANG, Xuan, Shenzhen Guangdong 518052 (CN)
(74) Representative: Maiwald Patent- und Rechtsanwaltsgesellschaft mbH
(86) International application number: PCT/CN2016/076328
(87) International publication number: WO 2017/156691

(57) **Abstract**

A shoe and a control method thereof. The shoe comprises: a shoe body (30) and a pressure sensor (40), controller (60), and prompting device (70) provided in the shoe body (30). The pressure sensor (40) senses pressures at different locations of the shoe body (30). When the pressure sensor detects that the pressure at a first position of the shoe body (30) exceeds a threshold, the controller (60) controls the prompting device (70) to prompt that the pressure at the first position has exceeded the threshold. The pressure detection and prompting actions provide effective monitoring of feet health.

## Description

### FIELD

The present disclosure relates to a field of wearable devices, and more particularly, to a shoe and a shoe control method.

### BACKGROUND

A shoe is used as a protection for foot, and is an essential commodity of human daily life. According to different functions and occasions, shoes may be in a variety of forms, such as high heels, sports shoes, sneakers and rain boots. With the improvement of people's living standards, more and more attentions have been paid to health, but the existing shoes cannot meet these health requirements due to its single function.

### SUMMARY

Accordingly, embodiments of the present disclosure provide a shoe with a health monitoring function and a method for controlling the same.

In embodiments of the present disclosure, a shoe is provided. The shoe includes: a shoe body, and a pressure sensor, a controller and a prompting device arranged on the shoe body, in which the pressure sensor is operated to detect a pressure of a position of the shoe body, and when the pressure sensor detects that a pressure in a first position of the shoe exceeds a threshold, the controller controls the prompting device to indicate a prompt of the pressure of the first position exceeding the threshold.

In embodiments of the present disclosure, a shoe control method is provided, and includes: detecting pressures at different positions of a shoe, determining whether a pressure of each position exceeds a threshold, indicating a prompt if a pressure of a first position exceeds the threshold.

A pressure caused by foot and a pressure distribution of the shoe may be monitored since pressure sensors are integrated in the shoe body. If the pressure at a position of the shoe exceeds a preset threshold, a prompt is sent by the prompting device to remind the user that the pressure of the position is too high, and thus the user may adjust foot posture according to the prompt. Therefore, foot pain or sprain due to improper foot posture can be avoided effectively and foot care of the user may be improved.

### BRIEF DESCRIPTION OF THE DRAWINGS

Drawings which are involved in the description of embodiments will be introduced below in brief for illustrating technical solutions in embodiments of the present disclosure, it will be appreciated that drawings described below are merely some implementations of the present disclosure, and other modifications can also be obtained by those who skilled in the art, without creative work.
Fig. 1 is a schematic diagram of a shoe according to an embodiment of the present disclosure.
Fig. 2 is a diagram of a functional module of a shoe in Fig. 1.
Fig. 3 is a schematic diagram of a sole of a shoe in Fig. 1.
Fig. 4 is a diagram of a shoe in Fig. 1 in a working condition.

### DETAILED DESCRIPTION

In order to make purposes, technical solutions and advantages of embodiments of the present disclosure more clear, reference will be made in detail to embodiments of the present disclosure with accompanying drawings.

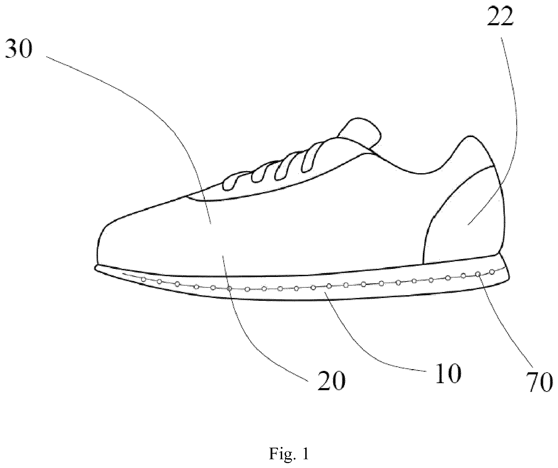

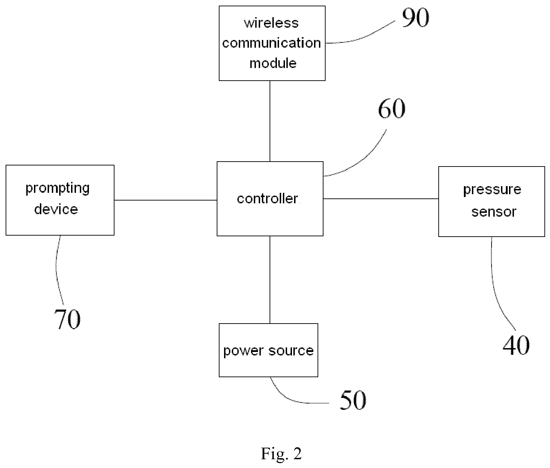

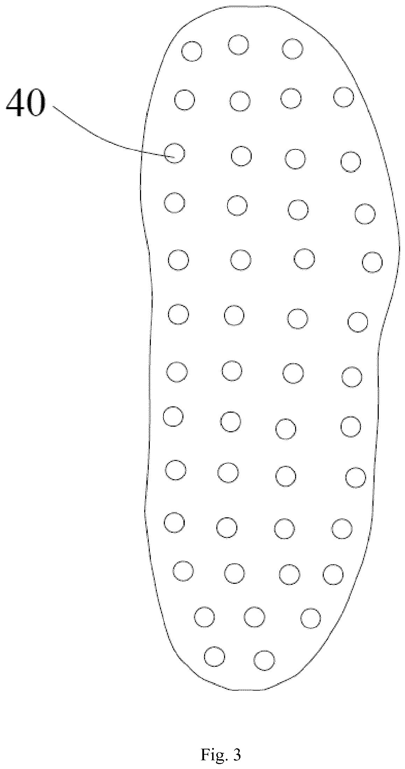

Figs. 1 to 3 show a shoe according to an embodiment of the present disclosure, and the shoe includes a shoe body 30 including a sole 10 and a vamp 20. A plurality of pressure sensors 40 arranged in an array is located on an upper surface of the sole 10. The pressure sensors 40 detect pressures at a plurality of positions of the sole 10 and the number and an arrangement of the pressure sensors 40 are not limited to the drawings, which can vary depending actual needs. For example, the pressure sensors 40 may be arranged in a loop, for example in a periphery of the sole 10, or in two lines from left to right or from front to rear of the sole 10 when a requirement for accuracy for detecting the pressure is not high.

Further, a power source 50, a controller 60 and a prompting device 70 are arranged on the sole 10. The controller 60 receives pressure data from the pressure sensors 40 and to determine whether the prompting device 70 should be controlled to indicate a prompt based on calculation of the pressure data. Alternatively, different pressure threshold data of different positions of the shoe may be stored in the controller 60. If a pressure of a position exceeds a preset threshold, the pressure of this position is determined to be overload by the controller 60 and a prompt is indicated by the prompting device 70. Different thresholds are set for different positions according to structures of feet. For example, a right side of the right foot bears more pressure than the left side, such that the pressure threshold set for a right side of a right shoe is more than that of a left side. The situation on the left foot is the opposite of the right one. The prompting device 70 reminds the user of an over-stressed position. In an embodiment of the present disclosure, the prompting device 70 includes a plurality of LED lights, which is arranged in the periphery of the sole 10 between an upper surface and a lower surface of the sole 10. In order to make the light emitted by the LED visible, the sole 10 may be made of a transparent material, such as polycarbonate (PC) and polymethyl methacrylate (PMMA). Alternatively, a transparent material may be only used for a portion of the sole 10 corresponding to the LEDs, and other portions of the sole 10 may be formed by non-transparent materials. A plurality of LEDs is arranged in the periphery of the sole 10, and the position with an overload pressure may be indicated precisely by emitting light from one of the LEDs selectively.

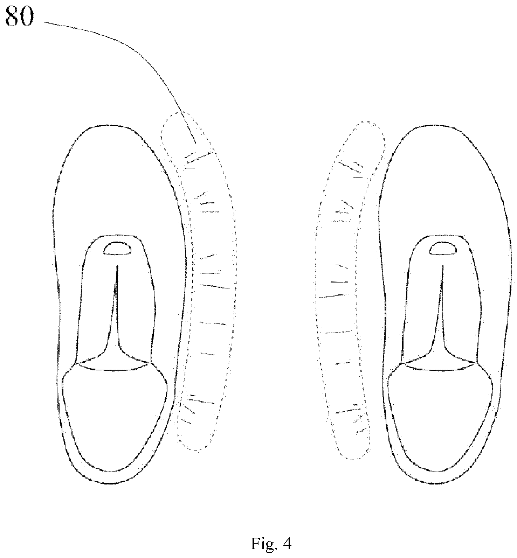

As shown in Fig. 4, when a user wears the shoes to exercise, the pressure sensor 40 continuously collects pressure data of the sole 10 and feeds it back to the controller 60 in real time. If a pressure of a first position exceeds a threshold (such as a left position of the right foot), the controller 60 controls the LED at a second position corresponding to the first position (such as a LED on the left side of the shoe) to emit light. The light may pass through the transparent portion of the sole 10 and irradiate on the ground to form a light band 80, thereby reminding the user that a certain portion of the foot bears an over high pressure and the posture of the foot needs to be changed. In an embodiment of the present disclosure, the first positon has a corresponding relationship to the second position, for example, the second position may be in an area below the first position or right below the first position, or be located on the side of the first position. Therefore, foot pain or sprain due to improper foot posture can be avoided effectively and foot care of the user may be improved.

In an embodiment of the present disclosure, it is possible that the controller 60 controls the LED to emit light once a pressure is detected by the pressure sensor 40. If the pressure of the position is in a normal range, a first color light is emitted. If the pressure of the position exceeds the pressure threshold, a second color light is emitted. Moreover, the first color and the second color are different. For example, the first color is green and the second color is red. Alternatively, LEDs may be set to have different light intensities. If the pressure of the position is in a normal range, light with a first intensity is emitted. If the pressure of the position exceeds the pressure threshold, light with a second intensity is emitted. Moreover, the first intensity is different from the second intensity. For example, the first intensity is low and the second intensity is high.

In an embodiment of the present disclosure, the prompting device is not limited to a LED, a prompt may be indicated in other types of light or other manners, such as a vibrator and a loudspeaker, as long as information of an over high pressure of a position can be provided to the user. A plurality of vibrators may be used and arranged in the periphery of the sole 10 as the LEDs. Once a pressure of a position is detected to exceed the threshold by the controller 60, a corresponding vibrator is vibrated to remind the user. Alternatively, a loudspeaker may be used and located in any position in the shoe. The loudspeaker may be controlled by the controller 60 to indicate a prompt which position (such as front, rear, left and right portions) bears an over high pressure.

It should be understood that in addition to the sole, pressure may also be formed on the instep of a foot. On this basis, pressure sensors 40 may be arranged at positions of a vamp 20 of the shoe. These sensors 40 may be working together with the pressure sensors 40 arranged on the sole 10, and thus improving the health care of the feet.

It can also be understood that the prompting device 70 can also be a flexible display provided on the upper surface of the vamp 20, which can directly show an outline of a foot on the display and indicate the position where the pressure is too high.

The aforementioned pressure detection function is usually required during exercise of the user, such that an exercise mode can be set for the shoe to realise above function, in other words, the pressure detection can be activated when the shoe is in the exercise mode. It should be understood that the shoe can also have other modes to meet different needs. In an embodiment of the present disclosure, the mode of the shoe may include an illumination mode, in which all the LEDs are emitting high in continuous manner to illuminate the surrounding area of the shoe, and thus allowing the user to walk or find objects in dark. In another embodiment, the light emitted is a white light with high intensity. In such an illumination mode, the pressure sensors 40 are turned off to save power. In a further embodiment of the present disclosure, the mode of the shoe may include a stage mode, in which the shoe has a built-in speaker, and the controller 60 controls the LED to emit light with different colors and intensities according to the frequency/volume of the sound of the music played by the speaker, and thus achieving an improved stage effect. Alternatively, a wireless communication module 90 can also be arranged in the shoe, which is operated to communicate with an external electronic device, and thus the built-in speaker can be omitted and the light can be controlled according to the music played by a speaker of the external electronic device. In such a stage mode, the pressure sensors 40 are turned off to save power. It should be understood that a force applied to a foot may vary in different types of movements, so the exercise mode may include at least one of a walking mode, a running mode, a basketball mode and a football mode. Specifically, the walking mode has a lower pressure threshold, a pressure threshold of the running mode is higher than that of the walking mode, and pressure thresholds of the basketball mode and the football mode are higher than that of the running mode.

A flexible touchpad 22 may be provided in a heel counter position of the vamp 20 and is operated to switch a plurality of modes of the shoe from one to another.

Because the flexible touchpad 22 is made of a transparent flexible material, it can be flexibly applied to the vamp 20 without affecting the overall appearance of the shoe. Alternatively, other types of operating elements can also be used to switch the modes of the shoe, such as a mechanical knob, a mechanical button, a hard touchpad and a toggle switch. The user can perform different gestures on the flexible touchpad 22 to switch the modes. For example, gestures of sliding up and down may switch modes of an exercise mode, an illumination mode and a stage mode, and gestures of sliding left and right may switch modes of a walking mode, a running mode, a basketball mode and a football mode.

In embodiments of the present disclosure, a shoe control method is provided. The method includes steps of: detecting a pressure distribution relating to different positions of the sole 10; determining whether a pressure of each position exceeds a threshold; indicating a prompt if a pressure of a position exceeds the threshold. The step of detecting a pressure distribution relating to different positions of the sole 10 is performed by the pressure sensor 40, the step of determining whether a pressure of each position exceeds a threshold is performed by the controller 60 and the step of indicating a prompt if a pressure of a position exceeds the threshold is performed by the prompting device 70. In another embodiment of the present disclosure, before the pressure distribution is detected, the plurality of modes of the shoe may be switched from one to another.

## Claims

1. A shoe, comprising:
a shoe body, and
a pressure sensor, a controller and a prompting device arranged on the shoe body,
wherein
the pressure sensor is operated to detect a pressure of a position of the shoe body, and
when the pressure sensor detects that a pressure in a first position of the shoe exceeds a threshold, the controller controls the prompting device to indicate a prompt of the pressure of the first position exceeding the threshold.

2. The shoe according to claim 1, wherein the shoe body comprises a sole and a vamp, and the pressure sensor is at least formed on the sole.

3. The shoe according to claim 2, wherein the prompting device comprises a light source arranged in the sole and a material of a position of the sole corresponding to the light source is transparent.

4. The shoe according to claim 3, wherein when the pressure of the first position exceeds the threshold, a light source of a second position is operated to provide a prompt, and the second position corresponds to the first position.

5. The shoe according to claim 2, further comprising a flexible touchpad located on the vamp and operated to switch a plurality of modes of the shoe from one to another.

6. The shoe according to claim 5, wherein pressure thresholds of a same position of the shoe in the plurality of modes are different.

7. The shoe according to claim 5, wherein the plurality of modes comprise an illumination mode, in which the prompting device is operated to emit light in a continuous manner.

8. The shoe according to claim 5, wherein the plurality of modes comprise a stage mode, in which the prompting device is operated to adjust a light color or a light intensity according to music being played.

9. A shoe control method, comprising:
detecting pressures at different positions of a shoe,
determining whether a pressure of each position exceeds a threshold,
indicating a prompt if a pressure of a first position exceeds the threshold.

10. The shoe control method according to claim 9, wherein indicating a prompt of the pressure of the first position exceeding the threshold is achieved by a light source emitting light at a second position corresponding to the first position.

11. The shoe control method according to claim 9, wherein indicating a prompt of the pressure of the first position exceeding the threshold is achieved by a vibrator vibrating at a second position corresponding to the first position.

12. The shoe control method according to claim 9, further comprising switching a plurality of modes of the shoe from one to another.

13. The shoe control method according to claim 12, wherein switching a plurality of modes of the shoe from one to another is prior to detecting pressures at different positions of the shoe.

14. The shoe control method according to claim 12, wherein pressure thresholds of a same position of the shoe in the plurality of modes are different.

15. The shoe control method according to claim 12, wherein switching a plurality of modes of the shoe from one to another is achieved by a flexible touchpad located in a heel counter position of the shoe.
